Europäisches Patentamt

**European Patent Office**

Office européen des brevets

(11) Publication number: **0 267 692 B1**

## EUROPEAN PATENT SPECIFICATION

(45) Date of publication of patent specification: **21.09.94**

(51) Int. Cl.5: **C12N 15/00**, C12P 21/02, A61K 37/64, C07K 7/10, G01N 33/68

(21) Application number: **87309087.2**

(22) Date of filing: **14.10.87**

The file contains technical information submitted after the application was filed and not included in this specification

(54) **Production of recombinant human PSTI.**

(30) Priority: **14.10.86 JP 245049/86**

(43) Date of publication of application:
**18.05.88 Bulletin 88/20**

(45) Publication of the grant of the patent:
**21.09.94 Bulletin 94/38**

(84) Designated Contracting States:
**AT BE CH DE ES FR GB GR IT LI LU NL SE**

(56) References cited:
**EP-A- 0 168 342
EP-A- 0 264 118
WO-A-86/03519
WO-A-88/03171**

**BIOCHEMICAL AND BIOPHYSICAL RE-SEARCH COMMUNICATIONS, vol. 132, no. 2, 30th October 1985, pages 605-612, Academic Press, Inc.; T. YAMAMOTO et al.:"Molecular cloning and nucleotide sequence of human pancreatic secretory trypsin in inhibitor (PSTI) cDNA"**

(73) Proprietor: **SHIONOGI SEIYAKU KABUSHIKI KAISHA trading under the name of SHIONOGI & CO. LTD.**
**1-8, Doshomachi 3-chome,
Chuo-ku
Osaka 541 (JP)**

(72) Inventor: **Ogawa, Michio**
**1-15-7, Uenozaka
Toyonaka-shi Osaka (JP)**
Inventor: **Matsubara, Kenichi**
**3-18-1-804, Yamadahigashi
Suita-shi Osaka (JP)**

(74) Representative: **Allard, Susan Joyce et al
BOULT, WADE & TENNANT
27 Furnival Street
London EC4A 1PO (GB)**

J. BIOCHEMISTRY, vol. 98, no. 3, 1985, pages 687-694; N. KIKUCHI et al.: "The multiplicity of human pancreatic secretory trypsin inhibitor"

CHEMICAL ABSTRACTS, vol. 100, 1984, page 235, abstract no. 63857p, Columbus,Ohio, US; M. OGAWA: "Purification and development of immunoassay for pancreatic enzymes and trypsin inhibitor, and their application to elucidation of pathogenesis of various pancreatic and pancreatic enzyme-producing diseases"

FEBS LETTERS, vol. 202, no. 2, June 1986, pages 373-377, Federation of European Biochemical Societies; J. DODT et al.: "Expression, secretion and processing of hirudin in E. coli using the alkaline phosphatase signal sequence"

GENE, vol. 40, 1985, pages 57-65, Elsevier Science Publishers; J. OBERTO et al.: "Expression of chicken egg white lysozyme by Saccharomyces cerevisiae"

J. BIOCHEM., vol. 102, 1987, pages 607-612; N. KIKUCHI et al.: "Production of recombinant human pancreatic secretory trypsin inhibitor by Escherichia coli"

CHEMICAL ABSTRACTS, vol. 104, 1986, page 380, abstract no. 49321k, Columbus,Ohio, US; M. OGAWA: "Pancreatic secretory trypsin inhibitor (PSTI)"

GENE, vol. 59, nos. 2-3, 1987, pages 151-159, Elsevier Science Publishers B.V.(Biomedical Division), Amsterdam, NL; Y. IZUMOTO et al.: "Expression of human pancreatic secretory trypsin inhibitor in Saccharomyces cerevisiae"

## Description

The invention relates to a process for the production of human pancreatic secretory trypsin inhibitor (hereinafter referred to as human PSTI), more particularly, it relates to a process for the production of human PSTI which comprises transforming a host, Saccharomyces cerevisiae, with a vector carrying a gene encoding human PSTI, and culturing the resultant transformant under appropriate conditions.

Trypsin inhibitors derived from the pancreas are divided into the pancreatic secretory trypsin inhibitor and basic pancreatic trypsin inhibitor. The former, PSTI, is found in kidney, lung, spleen, liver and brain as well as the pancreas, of mammals. The latter occurs in the pancreas and other organs of ruminants, but not in other mammals including humans.

Pubols et al., J. Biol. Chem., 249 2235, (1974), and Freinstein et al., Eur. J. Biochem., 43 569, (1973), independently separated and purified human PSTI from human pancreatic juice. Greene et al., Meth. Enzymol., 45 813, (1976), determined the primary structure of PSTI.

Human PSTI is a peptide consisting of 56 amino acid residues having a molecular weight of 6242. The SH groups of cysteinyl residues at positions 9 and 38, 16 and 35, and 24 and 56, respectively, form S-S bonds, and the peptide does not contain free SH groups. The amino acid sequence determined by Greene et al., (supra), is slightly different from the sequence determined by the present inventors in that 21 and 29 positions are respectively asparagine (Asn) and aspartic acid (Asp) according to the former, while they are, in contrast, aspartic acid and asparagine according to the latter.

Eddeland et al., Hoppe-Seyler's Z. Physiol. Chem., 359 671, (1978), and Kitahara et al., Biomed. J., 3 1119, (1979), independently established a radioimmunoassay system employing, as an antigen, human PSTI derived from pancreatic juice, which system enabled an immunological measurement of PSTI in blood. Yamamoto et al., Biochem. Biophys. Res. Commun., 132 695 (1985), determined the base sequence of a gene encoding human PSTI.

Autolysis in acute pancreatitis is caused by the action of proteolytic enzymes. A small amount of trypsin activated by an unknown reason appears to raise a chain-reaction type activation of trypsinogen and related zymogens. PSTI present in pancreatic acinus cells is secreted into the pancreatic juice together with other pancreatic enzymes and inhibits the activation of trypsin in the pancreatic duct. Part of the PSTI is transferred into the blood stream and remains therein as an escaped inhibitor (Cholecyst and Pancrea, 2 231, 1982).

The PSTI level in blood remarkably varies with pancreatic diseases, particularly acute pancreatic diseases. The retention time of high PSTI level in blood is longer than that of amylase. The variation of PSTI level in blood sharply reflects the magnitude of the damage in the pancreas irrespective of protease inhibitors (Cholecyst and Pancrea, 3 383, 1982). Accordingly, measurement of the PSTI level in blood permits diagnosis of pancreatic diseases and monitoring of the progress of the diseases.

Owing to recent progress in genetic engineering it has become eaier to obtain a large amount of a desired peptide by inserting a gene coding for the peptide into a vector and transforming a bacterial host such as Escherichia coli with the vector. However, expression in bacteria has several disadvantages. For instance, the expressed peptide in bacteria is accumulated within the cell, which may hinder the cell growth or repress the peptide production through a feedback system when overaccumulated. In addition, peptides which are small in size and expressed in bacteria are often decomposed by bacterial peptidases. Furthermore, recovery of the desired peptide requires the collection and destruction of the cultered cells and separation and purification of the peptide from the culture containing the destroyed cells. The culture contains various substances derived from the destroyed cells, part of which is toxic, and therefore, it is not easy to recover the desired peptide from the culture in a pure form.

In eucaryotic cells, secretory proteins as well as other proteins composing the cell wall are synthesized in the form of a precursor polypeptide having an additional amino acid sequence on one end of the protein, called a "signal peptide", which is necessary for the protein to pass through the cell membrane. The signal peptide is cleaved by peptidase present in the membrane when the precursor passes through the membrane, which gives an active and functional matured protein.

Attempts have been made to apply the above secretory system to the expression of desired proteins in order to obviate the aforementioned disadvantages inherent in expression in procaryotic cells. For this purpose, S. cerevisiae is known as a preferred host and has been employed for the expression of various peptides. See, for instance, Japanese Patent Publication (Kokai) Nos. 174396/1983, 70079/1985, 196093/1984, 205997/1984, 198980/1984 and 41487/1985.

Measurement of PSTI by radioimmunoassay has long been used for the diagnosis of pancreatic diseases and observation of the progress of the disease after treatment. Since human PSTI has been obtainable only from pancreatic juice, there has been some difficulty in establishing a good supply of the PSTI.

It has now been found that a substantial amount of human PSTI can be obtained by inserting a gene encoding human PSTI into a vector, transforming S. cerevisiae with the vector carrying the gene, and culturing the microorganism under appropriate conditions which allow for the expression of human PSTI.

Accordingly, the invention provides a process for the preparation of human PSTI which comprises transforming S. cerevisiae with an expression vector carrying a gene encoding human PSTI and culturing the resultant transformant under appropriate conditions which allow for the expression and secretion of human PSTI, centrifuging the culture to obtain a supernatant and recovering the secreted human PSTI from the supernatant.

In the accompanying drawings:

Figure 1 shows a base sequence coding for a human PSTI and a corresponding amino acid sequence.

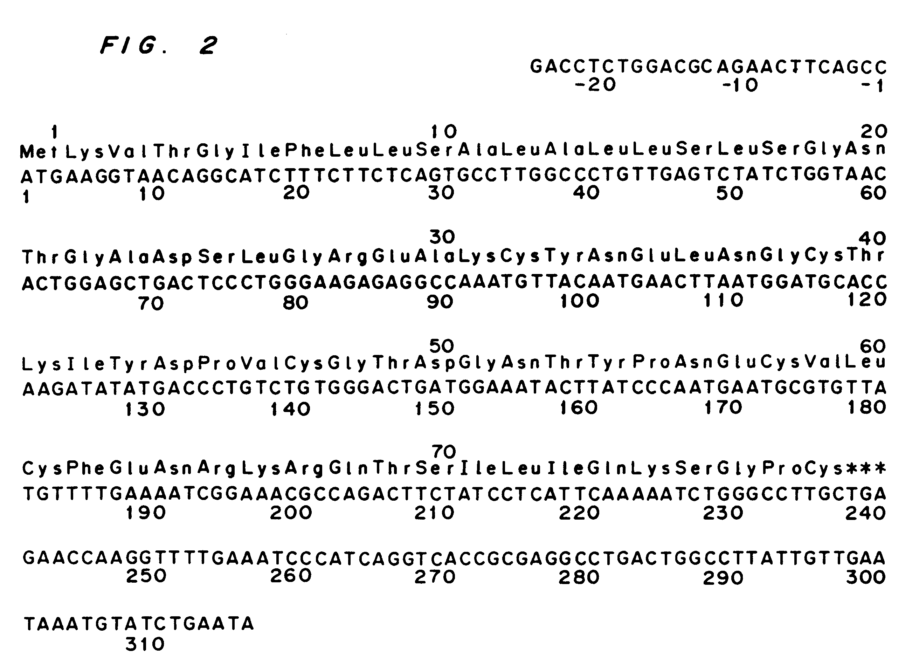

Figure 2 shows a base sequence coding for a human PSTI and a corresponding amino acid sequence which are accompanied by a leader sequence and a 3′ noncoding region.

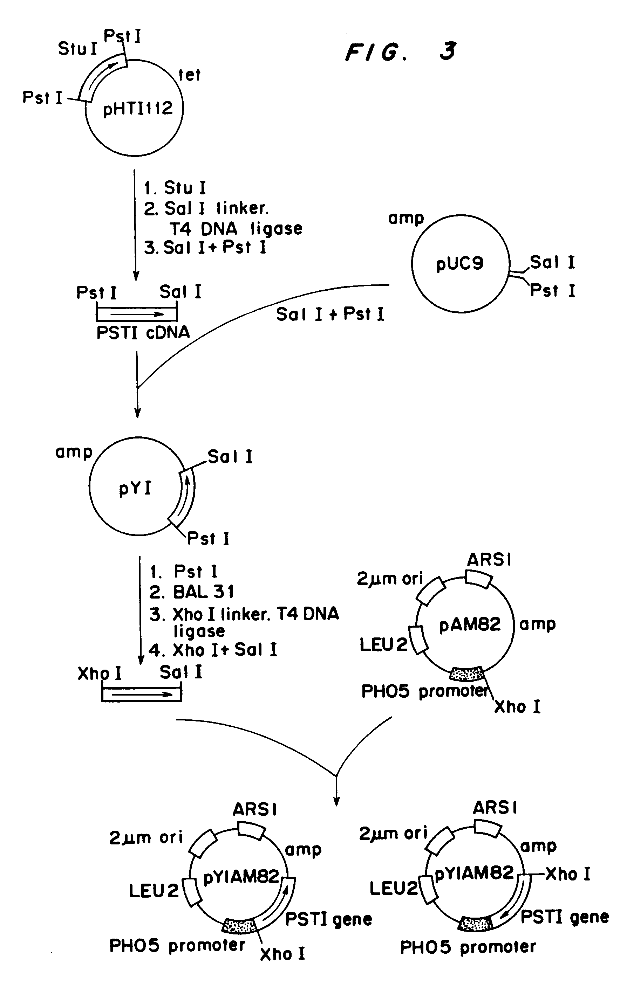

Figure 3 shows the construction of plasmid pYI AM8 comprising a gene coding for human PSTI.

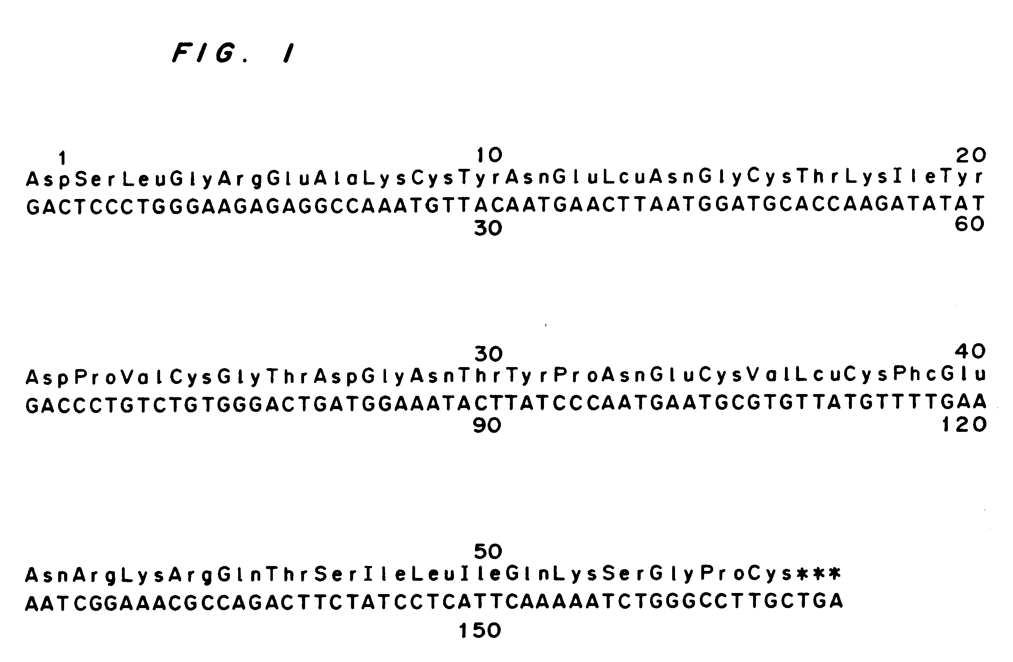

The base sequence of the natural gene encoding human PSTI has already been determined by Yamamoto et al. (supra). Accordingly, the DNA encoding human PSTI can be chemically synthesized, although it is also obtainable by reverse transcription of mRNA obtained from human tissue according to Yamamoto's paper. The DNA for huyman PSTI used in the present invention may be the one which has exactly the same base sequence as depicted in Figure 1 or its degenerate variants which encode the amino sequence shown in Figure 1.

For the purposes of the present invention, the term "gene coding for human PSTI" or the term "gene for human PSTI" usually means the structural gene which encodes human PSTI but it sometimes denotes a longer DNA sequence which comprises a promotor region, a signal peptide-encoding region, a terminator, and a polyadenylation signal, as well as the structural gene.

The vector into which the gene for human PSTI is to be inserted can be selected from, but is not limited to, those conventionally employed for the transformation of S. cerevisiae, such as YIp (e.g. YIp5, YIp32), pJDB2, YEp (e.g. YEp13), YRp (e.g. YRp7, YRp16), YCp (YCp19), cosmid vectors (e.g. pYc1, pYc2), vectors derived from 2μm DNA, and the like. Other known expression vectors may be used such as pAM82, pAT77, YEp52, AH5, AH9, AH10, AH21, pGPD-2, and the like.

The expression vector for human PSTI is constructed so that the structural gene may be positioned downstream of an appropriate promotor. If necessary, a terminator and polyadenylation signal are simultaneously inserted downstream of the structural gene. As the promotor, terminator and polyadenylation signal, there may be employed those associated with the expression of enolase, glyceraldehyde-3-phosphate dehydrogenase, hexokinase, pyruvate decarboxylase, phosphofructokinase, alcohol dehydrogenase, acid phosphatase, iso-cytochrome C, enzymes associated with glactose utilization, and the like. A combination of the above elements from different origins can also be employed. A DNA which codes for an appropriate signal peptide must be connected with the structural gene. The DNA coding for the signal peptide may be selected from, but is not limited to, those found in genomic DNA for α-factor, a-factor, acid phosphatase, invertase, or the like.

When the gene for human PSTI derived from human tissue is to be employed, it is convenient to clone the gene so that it may contain a signal peptide-coding gene, terminator and, if necessary, a polyadenylation signal, all associated with the structural gene for human PSTI. The resultant gene is then placed downstream of a suitable promotor in an expression vector to obtain a vector capable of expressing human PSTI. As stated before, the structural gene for human PSTI can be chemically synthesized because it is relatively short in length.

S. cerevisiae is the preferred host for the expression of human PSTI. Examples of S. cerevisiae hosts are, among others, Saccharomyces cerevisiae KM-46 (ATCC No. 26923), H-42 (ATCC No. 26922), BH-64-1A (ATCC No. 28339), and the like.

Expressed human PSTI can be conventionally purified from the culture by, for example, chromatography, affinity chromatography, centrifugation, or a combination thereof.

The following detailed Example is presented by way of illustration.

EXAMPLE

A. Cloning of cDNA encoding human PSTI

Total RNAs were obtained from human pancreas which had been frozen in liquid nitrogen and stored at -70°C by guanidine-phenol/chloroform method (Gene, 28 263-270, 1984). Salivary gland, stomach, or liver can be used in place of pancreas. Poly(A) RNA was separated from the total RNAs by repeated oligo(dT) cellulose column chromatography. In accordance with the Schibler's method (Cell, 15 1495-1509, 1978), double stranded cDNA was prepared using the poly(A) RNA. The resultant cDNA was treated with S1 nuclease following the teaching of Roychondhury et al. (Methods in Enzymology, 65 43-62), and subsequently added with dC elongation by the use of terminal transferase. The resultant DNA was annealed with pBR322 which had been digested with restriction enzyme PstI and then added with dG elongation. The annealed mixture was used to transform E.coli K12 HB101 in accordance with the Dagert's method (Gene, 6 23-28, 1979). The transformant was selected on an agar plate containing tetracycline, and a cDNA library was prepared therefrom.

A series of oligonucleotide probes each consisting of 14 base pair were prepared as shown below by phosphotriester method (Nucleic Acids Research, 10, 4467-4482, 1982) on the basis of the knowledge of the amino acid sequence of human PSTI (Methods in Enzymology, 45, 813-825).

```
            8    9   10   11   12
          Lys-Cys-Tyr-Asn-Glu
          5'                    3'
    mRNA  AAA  UGU  UAU  AAU  GAA
           G    C    C    C    G
          3'                    5'
   Probe  TTT  ACA  ATA  TTA  CT
           C    G    G    G
```

The prepared oligonucleotides, after purification by HPLC, were labelled with $^{32}$P at 5$'$ terminal using T4 polynucleotide kinase and employed as probes for hybridization described below.

About 1800 colonies from the aforementioned cDNA library were transferred onto a nylon filter, and the filter was subjected to a hybridization process with these probes, whereby 9 positive clones were obtained. A clone which appeared to contain part of cDNA encoding human PSTI was selected by restriction enzyme analysis and designated pHT19. The cDNA inserted in pHT19 was purified and employed as a second probe for further screening of cDNA. This provided a plasmid bearing cDNA insert of 431bp, which was designated pHT112 (Yamamoto et al., Biochem. Biophys. Res. Commun., 605-612, 132 1985). Using the cloned cDNA from pHTI112, a full-length base sequence of PSTI gene was determined by M13 method (Proc. Natl. Acad. Sci. U.S.A., 74 560-564, 1977).

## B. Construction of Expression Vector

Plasmid pHTI112 (20μg) was digested with 20 units of a restriction enzyme StuI in 400μl of a StuI buffer solution (10mM Tris-HCl, pH 8.0, 7mM MgCl₂, 100mM NaCl, 7mM 2-mercaptoethanol, 0.01% bovine serum albumin) at 37°C for 60 minutes. After reaction, the mixture was extracted with phenol/chloroform, and then ethanol-precipitated by addition of 1/10 volume of 3M sodium acetate, pH 5.3, and 2.5 volumes of ethanol. The precipitate was dried under slight vacuum, dissolved in water, and employed in subsequent reaction. The phenol/chloroform extraction and ethanol precipitation were routinely conducted when an enzyme treatment was carried out in the following procedures.

The resultant plasmid DNA was linked with SalI linker, which is self-complementary oligonucleotide having the sequence pdGGTCGACC, in order to add thereto SalI restriction site which is helpful for insertion of the desired DNA into the SalI site of plasmid pUC9 in the later stage. Thus, 2μg of StuI-digested pHTI112 was combined with 32 picomoles of 5$'$-phosphorylated synthetic oligonucleotide pdGGTCGACC using 350 units of T4 DNA ligase in 40μl of a T4 DNA ligase buffer (66mM Tris-HCl, pH 7.4, 1.0mM ATP, 66mM MgCl₂, 10mM dithiothreitol, 0.01% bovine serum albumin) at 18°C for 12 hours. The mixture was heated at 70°C for 10 minutes for terminating the ligase reaction, and used for the transformation of E. coli K12 HB101 to obtain the desired transformant.

The resultant plasmid (20μg) was then digested with SalI. For this purpose, the reaction mixture was added with the following components at designated concentrations to make a SalI buffer:

10mM Tris-HCl, pH7.5

7mM MgCl₂

175mM NaCl

0.2mM EDTA

7mM 2-mercaptoethanol

EP 0 267 692 B1

0.01% bovine serum albumin

The mixture was treated with 10 units of SalI at 37°C for 60 minutes. The SalI-digested DNA was further digested with PstI, and the DNA fragment which comprises PSTI structural gene and PstI and SalI cohesive ends was separated. Thus, 20μg of SalI-digested DNA was treated with 32 units of a PstI in 200 μl of a PstI buffer (10mM Tris-HCl, pH 7.5, 10mM MgCl₂, 50mM ammonium sulfate, 0.01% bovine serum albumin) at 37°C for 60 minutes. The mixture was subjected to 5% polyacrylamide gel electrophoresis and stained by ethidium bromide to locate the gel containing the desired fragment by U.V. light. The relevant gel was cut and recovered, and then homogenized in 10mM Tris-HCl, pH 8.0, containing 1mM EDTA. The supernatant was treated with ethanol to recover the desired DNA fragment. The PstI-SalI fragment containing PSTI structural gene was inserted into PstI-SalI digested plasmid pUC9 by the use of T4 DNA ligase. Thus, 0.1μg of PstI-SalI digested pUC9 was combined at 18°C for 12 hours with 0.5μg of the PstI-SalI fragment in the presence of 350 units of T4 DNA ligase in 30μl of T4 DNA ligase buffer. The mixure was used to transform E. coli HB101 according to the Mandel and Higa's method (J. Mol. Biol., 53 154, 1970). The transformant was selected on an agar plate containing ampicilline. Several ampicillin-resistant colonies were selected, and plasmid DNA was separated. The presence of the desired fragment was confirmed by restriction cleavage pattern analysis. The resultant plasmid was designated pYI.

The palsmid pYI was digested with PstI and 5′ noncoding region of the human PSTI gene was deleted by BAL 31 nuclease. XhoI linker, which comprises self-complementary oligonucleotide having a sequence of pdCCTCGAGG, was attached to the digested linear plasmid to provide it with XhoI restriction site which is helpful for the insertion of the cDNA into the XhoI site of pAM82.

Thus, plasmid pYI was digested with PstI and the digested pYI (3μg) was treated with 2.6 units of BAL 31 nuclease at 30°C for one to ten minutes in 75μl BAL 31 nuclease buffer (20mM Tris-HCl, pH 8.0, 12mM CaCl₂, 12mM MgCl₂, 1mM EDTA, 0.6M NaCl). In a similar manner as before, XhoI linker was ligated to the BAL 31-digested pYI using T4 DNA ligase. The reaction mixture was used to transform E.coli HB101 according to the Mandel and Higa's method. Transformants were selected on an agar plate containing ampicillin, and plasmid DNA was separated from the transformant colonies. The extent of nucleotides deletion by BAL 31 nuclease was confirmed by determination of the base sequence by means of the dideoxy method using M13 DNA sequencing primer according to the Sanger's method (J. Mol. Biol., 143,161-178, 1980). A plasmid, in which 5′ noncoding region has been deleted up to 25 bp upstream from the ATG codon, was selected for subsequent construction.

The selected plasmid DNA was digested with XhoI and SalI, and a fragment comprising PSTI structural gene and XhoI and SalI cohesive ends was separated. Thus, the plasmid DNA (10μg) was treated with 24 units of XhoI at 37°C for 60 minutes in 100μl of a XhoI buffer (10mM Tris-HCl, pH 7.5, 7mM MgCl₂, 100mM NaCl, 7mM 2-mercaptoethanol). The XhoI-digested DNA was subsequently digested with SalI, and the resultant mixture was subjected to 5% polyacrylamide gel electrophoresis to recover the desired DNA fragment.

An expression vector derived from S. cerevisiae, pAM82 (Miyanohara et al., Proc. Natl. Acad. Sci., U.S.A. 80 1-5, 1983, FERM-BP 313, converted from Bikoken No. 6668), was digested with XhoI and ligated, in the manner as described below, with the XhoI-SalI restriction fragment which contained PSTI structural gene.

XhoI-digested pAM82 (0.5μg) was combined with the XhoI-SalI fragment (2μg) in the presence of 350 units of T4 DNA ligase at 18°C for 12 hours in 30μl of T4 DNA ligase buffer. The reaction mixture was used to transform E. coli HB101 according to the Mandel and Higa's method. Transformants were selected on an agar plate containing ampicillin and the plasmid DNA was prepared from the transformed colonies. The presence of the desired fragment and orientation of the inserted fragment were confirmed by restriction cleavage pattern.

## C. Expression and Purification of Human PSTI

Isolated plamid DNA was used to transform S.cerevisiae AH22 in accordance with the teaching of Hinnen et al. (Proc. Natl. Acad. Sci., U.S.A. 75 1929-1933, 1978). Transformants were selected on an overlayed agar plate containing histidine. The resultant leucine-independent cell, designated as pYIAM82/AH22, was employed in subsequent experiments.

pYIAM82/AH22 clone, which has been confirmed to contain the expression plasmid, carries PH05 promotor which permits easy switching on and off of the expression depending on the presence or absence of inorganic phosphate in the culture medium. (Nakao et al., Molec. Cell. Biol., 6 2613-2623, 1986). The clone obtained above was employed in the subsequent expression procedure.

6

The following experiments were conducted substantially in accordance with the Miyanohara's method (Proc. Natl. Acad. Sci., U.S.A. 80 1-5, 1983).

i) Preparation of Culture Medium

Burkholder minimal medium containing 1.5g of potassium phosphate/L and the medium containing no phosphate (1.5g potassium chloride/L) were employed. Two hundred and fifty ml of a 4-fold concentrated stock solution of the phosphate-containing solution (P + medium) or the phosphate-free solution (P-medium), as listed below in Table 1, 1ml of vitamines stock solution, as listed below in Table 2, 20g of glucose, and 2g of asparagine were combined together in sufficient water to make a total volume of one liter.

After stirring, 50mg of histidine hydrochloride was added. By addition of 0.2N NaOH, the P + medium was rendered to pH 6.0 and the P-medium to pH 7.5. The P + medium and P-medium was autoclaved at 120°C for 10 minutes and at 10°C for 10 minutes respectively.

### Table 1  4-fold concentrated stock solution

| | |
|---|---|
| P+ KH$_2$PO$_4$ | 6g |
| P- KCl) | 6g |
| MgCl$_2$·7H$_2$O | 2g |
| CaCl$_2$·2H$_2$O | 1.32g |
| 0.05% KI | 0.8ml |
| Metal element (x10$^4$) (Table 1')* | |
| B, Mn, Zn, Cu | 0.2ml |
| Fe | 0.2ml |
| Mo | 0.2ml |
| Water | q.l. |
| | 1000ml |

The mixture was not autoclaved.

### *Table 1'  Preparation of stock solution of metal elements

| | |
|---|---|
| H$_3$BO$_3$ | 30mg |
| MnSO$_4$·7H$_2$O | 50mg |
| ZnSO$_4$·7H$_2$O | 150mg |
| CuSO$_4$·5H$_2$O | 20mg |
| Sterile distilled water | q.l. |
| | 50ml |
| Na$_2$MoO$_4$·2H$_2$O | 100mg |
| Sterile distilled water | q.l. |
| | 50ml |
| FeCl$_2$·6H$_2$O | 125mg |
| sterile distilled water | q.l. |
| | 50ml |

The above solutions were not autoclaved.

## Table 2  Vitamines stock solution

| | |
|---|---|
| Vitamine B$_1$ | 20mg |
| Pyridoxine | 20mg |
| Nicotinic acid | 20mg |
| Calcium pantothenate | 20mg |
| Biotin | 0.2mg |
| Inositol | 1g |
| Water | q.l. |
| | 100ml |

**The solution was sterilized by Millipore filtration.**

ii) Induction of PSTI Expression

The colonies grown on a plate were transferred to 10ml of P + Medium, and cultured with agitation at 30°C for two days. Part of the culture (0.2ml) was used to cultivate in P-medium and the rest was subjected to differential analysis as described below.

The above culture (0.2ml) from the P + medium was added to 10ml of P-medium containing 0.05ml of 2M Tris-HCl, pH 7.0, and the mixture was cultured at 30°C for two days and used as a P-medium sample.

iii) Fractionation of culture and Determination of PSTI.

Cultured P + medium and cultured P-medium were fractionated following Chart I described below. Each medium was centrifuged at 1500xg for 5 minutes. Cell precipitates were recovered and the supernatant was stored as a secretion fraction.

To the cell precipitates obtained above was added 1ml of spheroplasting buffer (1.2M sorbitol, 50mM phosphate buffer, pH 7.2, 300-500µg/ml of Zymolyase (60,000 units, Seikagaku Kogyo Co., Ltd.), and 14mM 2-mercaptoethanol) containing 1mM phenylmethylsulfonyl fluoride (PMSF), which is a protease inhibitor, and the resulting mixture was stirred by Vortex (Scientific Industries Inc.) and incubated at 30°C for one hour. The centrifugation of the mixture at 2500xg for 5 minutes gave periplasm components in the supernant and spheroplast components in the precipitate.

To the spheroplast precipitate thus obtained was added 1ml of a lysis buffer (0.2% Triton, 10mM phosphate buffer, pH 7.2, 150mM NaCl, 1mM PMSF), and the mixture was vortexed and allowed to react on ice for one hour to effect the lysis of the spheroplast. The centrifugation of the lysate at 15,000xg for 20 minutes yielded a cell extract as the supernatant.

## Chart 1  Fractionation of S. cerevisiae culture

S. cerevisiae culture

|

Centrifugation (2500xg, 5min.)

|

Supernatant
(Secretory Fraction)    Precipitate (Cells)

|

Spheroplasting Buffer
(containing 1mM PMSF)

|

Vortex (30°C, 1hr)

|

Centrifugation (2500xg, 5min.)

|

Supernatant               Precipitate
(Periplasm Layer)         (Spheroplast)

|

Lysis Mixture

|

Vortex (1hr stand at 0°C)

|

Centrifugation
(15000xg, 20min.)

|

Supernatant               Precipitate
(Cell Extract)

The PSTI production was measured by immunoassay on the secretory fraction, the periplasm fraction, and the cell extract fraction. Table 3 below lists the results of the assay.

## Table 3  PSTI Content in Each Fraction

| Fractions | PSTI Content (ng/ml) | |
|---|---|---|
| | P-Medium | P+Medium |
| Secretory Fraction | 1619 | 0.7 |
| Periplasm Fraction | 596 | 8.1 |
| Cell Extract Fraction | 321 | 0 |

10

Table 3 shows that a large amount of the produced PSTIs are secreted extracellularly.

iv) Cultivation in large scale

On the basis of the above results, the cultivation was conducted in a large scale (3L), and PSTI was recovered and purified in the manner as described below.

The colony on the plate was used to inoculate 10ml of P+medium and cultured at 30°C for 2 days with agitation. Five ml of the resultant culture was added to 100ml of P+medium and grown at 30°C for 2 days with agitation. Sixty ml of the culture was added to 3L of P-medium and cultivated with agitation at 30°C for 2 days. The culture was centrifuged at 7000xg for 10 minutes to obtain the secretory fraction in the supernatant.

v) Purification

The secretory fraction (700ml) was adjusted to pH 8.0 by addition of 1N sodium hydroxide, and loaded onto a bovine trypsin-CH-sepharose 4B column (1x3.2cm). After the column was successively washed with 0.05M Tris-HCl containing 0.5M NaCl, pH 8.0, and distilled water, PSTI was eluted with 10mM HCl. The eluted product was lyophilized to obtain 0.69mg of purified PSTI.

The resultant human PSTI (12$\mu$g) was placed into a test tube (10x90mm) and hydrolyzed under reduced pressure at 110°C for 24 hours after addition of 50$\mu$l of 4M methanesulfonic acid containing 0.2% 3-(2-aminoethyl)indole. Amino acid analysis was conducted using Hitachi Model 835 Amino Acid Analyzer. The resulting amino acid composition given in Table 4 was entirely consistent with that of natural human PSTI.

The sequence of three amino acid residues at N-terminal was determined as Asp-Ser-Leu according to the Edman's method, which is the modification of Iwanaga's method (Eur. J. Biochem. 8 189-199, 1969). The sequence of N-terminal was also consistent with that of natural human PSTI. In addition, the product of the invention inhibited bovine trypsin activity stoichiometrically, i.e., at 1:1 ratio. Finally, the immunoreactivity of the human PSTI of the invention with the antibody to natural human PSTI (rabbit antiserum polyclonal) was consistent with that of the natural PSTI, when compared using various diluted samples.

## Table 4

| Amino Acid | Found | Theoretical |
|---|---|---|
| Aspartic acid | 7.9 | 8 |
| Threonine | 3.7 | 4 |
| Serine | 2.5 | 3 |
| Glutamic acid | 6.2 | 6 |
| Proline | 3.1 | 3 |
| Glycine | 5.2 | 5 |
| Alanine | 1.1 | 1 |
| Cystine | 2.7 | 3 |
| Valine | 2.1 | 2 |
| Methionine | 0.0 | 0 |
| Isoleucine | 2.9 | 3 |
| Leucine | 4.0 | 4 |
| Tyrosine | 2.9 | 3 |
| Phenylalanine | 1.1 | 1 |
| Lysine | 3.9 | 4 |
| Histidine | 0.0 | 0 |
| Tryptophan | 0.0 | 0 |
| Arginine | 3.0 | 3 |

In light of the above, the invention includes a recombinant human PSTI. Also included is a polypeptide having the activity of human PSTI and the sequence shown in Figure 1 and being substantially free of other peptides, which polypeptide may have a leader sequence. The invention includes, of course, DNAs encoding the aforesaid, taking full acount of allelic equivalents and the degeneracy of the genetic code.

The invention further includes a PSTI expression vector for use in Saccharomyces cerevisiae and carrying a gene encoding human PSTI or a DNA sequence as above.

Also generally provided by the invention is the use of a recombinant human PSTI in in vitro diagnosis or disease monitoring.

**Claims**

1. A process for the production of human PSTI which comprises transforming Saccharomyces cerevisiae with an expression vector bearing a gene encoding human PSTI and if not included in the gene a signal peptide-encoding region therefor and culturing the resultant transformant under appropriate conditions which allow for the expression and secretion of human PSTI, centrifuging the culture to obtain a supernatant and recovering the secreted human PSTI from the supernatant.

2. A process of claim 1, wherein the gene encodes the amino acid sequence depicted in Figure 1 or Figure 2 of the accompanying drawings or wherein the gene corresponds to the base sequence depicted in Figure 1 or Figure 2 of the accompanying drawings.

**Patentansprüche**

1. Verfahren zur Herstellung von menschlichem PSTI, bei dem man Saccharomyces cerevisiae mit einem Expressionsvektor transformiert, der ein menschliches PSTI-codierendes Gen trägt und, falls in dem Gen nicht enthalten, einen Signalpeptid-codierenden Bereich dafür, und die erhaltene Transformante unter geeigneten Bedingungen züchtet, die die Expression und Sezernierung von menschlichem PSTI gestatten, die Kultur zentrifugiert, um einen Überstand zu erhalten, und das sezernierte menschliche PSTI aus dem Überstand gewinnt.

2. Verfahren nach Anspruch 1, bei dem das Gen die in Figur 1 oder Figur 2 der beigefügten Zeichnungen abgebildete Aminosäuresequenz codiert, oder wobei das Gen der in Figur 1 oder Figur 2 der beigefügten Zeichnungen abgebildeten Basensequenz entspricht.

**Revendications**

1. Procédé de préparation d'un PSTI humain qui comprend le fait de transformer Saccharomyces cerevisiae avec un vecteur d'expression portant un gène codant pour le PSTI humain, et s'il n'est pas inclus dans le gène, une région codant pour le peptide signal pour celui-ci, et de cultiver le transformant obtenu dans des conditions appropriées permettant l'expression et la sécrétion de PSTI humain, de centrifuger la culture pour obtenir un liquide surnageant et de récupérer le PSTI humain secrété dans le liquide surnageant.

2. Procédé selon la revendication 1, dans lequel le gène code pour la séquence d'aminoacides décrite dans la Figure 1 ou dans la Figure 2 des dessins annexés, ou dans lequel le gène correspond à la séquence de bases représentée dans la Figure 1 ou la Figure 2 des dessins annexés.

EP 0 267 692 B1

## FIG. 1

```
 1                              10                               20
AspSerLeuGlyArgGluAlaLysCysTyrAsnGluLcuAsnGlyCysThrLysIleTyr
GACTCCCTGGGAAGAGAGGCCAAATGTTACAATGAACTTAATGGATGCACCAAGATATAT
                               30                               60
```

```
                               30                               40
AspProValCysGlyThrAspGlyAsnThrTyrProAsnGluCysValLcuCysPhcGlu
GACCCTGTCTGTGGGACTGATGGAAATACTTATCCCAATGAATGCGTGTTATGTTTTGAA
                               90                              120
```

```
                               50
AsnArgLysArgGlnThrSerIleLeuIleGlnLysSerGlyProCys***
AATCGGAAACGCCAGACTTCTATCCTCATTCAAAAATCTGGGCCTTGCTGA
                              150
```

FIG. 2

GACCTCTGGACGCAGAACTTCAGCC
-20       -10       -1

1                            10                           20
MetLysValThrGlyIlePheLeuLeuSerAlaLeuAlaLeuLeuSerLeuSerGlyAsn
ATGAAGGTAACAGGCATCTTTCTTCTCAGTGCCTTGGCCCTGTTGAGTCTATCTGGTAAC
       10           20           30           40           50           60

                         30                           40
ThrGlyAlaAspSerLeuGlyArgGluAlaLysCysTyrAsnGluLeuAsnGlyCysThr
ACTGGAGCTGACTCCCTGGGAAGAGAGGCCAAATGTTACAATGAACTTAATGGATGCACC
       70          80          90         100         110         120

                         50                           60
LysIleTyrAspProValCysGlyThrAspGlyAsnThrTyrProAsnGluCysValLeu
AAGATATATGACCCTGTCTGTGGGACTGATGGAAATACTTATCCCAATGAATGCGTGTTA
     130         140         150         160         170         180

                         70
CysPheGluAsnArgLysArgGlnThrSerIleLeuIleGlnLysSerGlyProCys***
TGTTTTGAAAATCGGAAACGCCAGACTTCTATCCTCATTCAAAAATCTGGGCCTTGCTGA
     190         200         210         220         230         240

GAACCAAGGTTTTGAAATCCCATCAGGTCACCGCGAGGCCTGACTGGCCTTATTGTTGAA
     250         260         270         280         290         300

TAAATGTATCTGAATA
     310

EP 0 267 692 B1

FIG. 3